# EUROPEAN PATENT SPECIFICATION

(11) **EP 3 749 339 B1**
(45) Date of publication and mention of the grant of the patent: **03.08.2022**
(21) Application number: 19714777.0
(22) Date of filing: 30.01.2019
(51) Int. Cl.: A61K 35/612, A61K 38/01, A23K 10/22, A23K 20/147, A23J 3/34, A23L 33/18, A61K 31/131, A61K 31/198, A61K 31/401, A61K 31/405, A61K 33/00, A61K 33/06, A61K 33/16, A23L 33/10, A61K 35/60, A61K 31/04, A61K 31/4172

(54) **MARINE PROTEIN HYDROLYSATE WITH LOW FLUORIDE AND TRIMETHYLAMIN CONTENT**
MEERESPROTEINHYDROLYSATE MIT NIEDRIGEN FLUORID UND TRIMETHYLAMIN GEHALT
HYDROLYSAT DE PROTÉINES MARINES AYANT UN FAIBLE TAUX DE FLUOR ET DE TRIMÉTHYLAMINE

(30) Priority: 30.01.2018 US 201862623658 P
(43) Date of publication of application: 16.12.2020
(60) Divisional application: 22179865.5
(73) Proprietor: AKER BIOMARINE ANTARCTIC AS, 8340 Stamsund (NO)
(72) Inventor: ELLEGÅRD, Katrine, Hvid, 8340 Stamsund (NO); BAGGER, Christian, Lorentz, 8340 Stamsund (NO); MYHREN, Finn, 8340 Stamsund (NO); WIKLUND, Erik, Digman, 8340 Stamsund (NO)
(74) Representative: Onsagers AS
(86) International application number: PCT/IB2019/000118
(87) International publication number: WO 2019/150197

(56) References cited:
- WO-A1-2010/030193
- WO-A1-2014/184655
- CN-A- 103 238 723
- CN-A- 103 960 699
- CN-A- 104 186 911
- CN-A- 104 195 206
- CN-A- 104 232 717
- CN-A- 104 263 787
- CN-A- 106 010 783
- FR-A1- 2 927 336
- Li Laihao ET AL: "STUDIES ON THE EXTRACTION OF FISH PROTEIN CONCENTRATE (FPC) BY SOLVENT EXTRACTION", Asia Pacific Fishing Comission, Meeting report, 1 January 1998 (1998-01-01), pages 114-119, XP055589785, Retrieved from the Internet: URL:http://www.fao.org/3/a-bm148e.pdf [retrieved on 2019-05-17]
- J F Bergmann Verlag ET AL: "Lebensmittel- Untersuchung und-Forschung A Functional Protein Concentrate (FKPC) from Antarctic Krill (Euphausia superba, Dana 1850)* ** Preparation, Chemical Composition and Functional Properties", Z Lebensm Unters Forsch, 1 January 1981 (1981-01-01), XP055589787, Retrieved from the Internet: URL:https://link.springer.com/content/pdf/ 10.1007/BF01127671.pdf [retrieved on 2019-05-17]

## Description

### FIELD OF THE INVENTION

The present invention provides krill protein hydrolysates which may be used in pharmaceuticals, nutraceuticals, functional foods, foods, beverages and animal feeds as well as methods for making such hydrolysate.

### BACKGROUND OF THE INVENTION

There is an increasing interest in bioactive peptides from marine secondary products, as they offer a great potential for incorporation into functional food and for medical purposes. Bioactive peptides from marine sources have been found to display a wide range of physiological functions including antioxidative, antihypertensive, antimicrobial, immunomodulatory, anticancer and diabetes 2 effects among others. Most of the research has focused on preparation and characterization of hydrolysates for commercial use in health and functional foods.

Krill hydrolysates for use in marine feeds and as human supplements have been described. See, e.g., WO2010030193; WO2013102792; Kolkovski et al., J. World Aqua. Soc., Volume 31, Issue 1 (2000) pp. 81-88. These krill hydrolysates are generally produced from sources that contain high amounts of lipids.

CN 103 960699 discloses methods for producing a krill peptide-ferrous-zinc-calcium complex. Products and intermediate products obtained by said method are also being disclosed.

CN 104 195 206 discloses a process involving hydrolysis of a defatted krill powder, defluorination, treatment for removing TMAO/TMA/TVN, desalting and spray drying.

CN 104 263 787 discloses a process involving hydrolysis of a defatted krill powder, simulated moving bed (SMB) technology for removing fluoride and ash, and spray drying.

WO 2010/030193 discloses a process for reducing fluoride content in krill protein hydrolysates by separating the solids after proteolytic hydrolysis of milled krill.

WO 2014/184655 describes a process for making krill phospholipid protein concentrate with low fluoride content.

CN 103 238 723 describes a process for obtaining low-fluoride krill protein hydrolysate.

### SUMMARY OF THE INVENTION

The present invention provides krill protein hydrolysates which may be used in pharmaceuticals, nutraceuticals, functional foods, foods, beverages and animal feed as well as methods for making such protein hydrolysates.

The krill protein hydrolysate of the present invention having a protein content of greater than 85% on a dry weight basis, less than 5% fat on a dry weight basis, a fluoride content of from 0.1 to 200 mg/kg hydrolysate on a dry weight basis; a TMAO (trimethylamine N-oxide) content of from 0.1 to 200 mg N/100 g hydrolysate on a dry weight basis; a TMA (trimethylamine) content of from 0.1 to 200 mg N/100 g hydrolysate on a dry weight basis; a TVN (total volatile nitrogen) content of from 0.1 to 200 mg N/100 g hydrolysate on a dry weight basis; a sodium content of from 0.01 to 4.0g/100g hydrolysate on a dry weight basis; and a calcium content of from 100 to 25,000 mg/kg hydrolysate on a dry weight basis.

The krill protein hydrolysate of the present invention may also have one or more of the following properties:
g. a lysine content of from 6.30 to 10.30 g lysine/100g total amino acids;
h. a threonine content of from 3.04 to 7.04 g threonine/100g total amino acids;
i. an isoleucine content of from 3.39 to 7.39 g isoleucine/100g total amino acids;
j. a leucine content of from 6.27 to 10.27 g leucine/100g total amino acids;
k. a histidine content of from 0.94 to 3.94 g histidine/100g total amino acids;
l. a phenylalanine content of from 2.76 to 6.76 g phenylalanine/100g total amino acids;
m. a tyrosine content of from 2.39 to 6.39 g tyrosine/100g total amino acids;
n. a valine content of from 3.69 to 7.69 g valine/100g total amino acids;
o. an alanine content of from 3.76 to 7.76 g alanine/100g total amino acids;
p. an arginine content of from 3.90 to 7.90 g arginine/100g total amino acids;
q. an aspartic acid/asparagine content of from 9.68 to 13.68 g aspartic acid and asparagine/100g total amino acids;
r. a glutamic acid/glutamine content of from 11.54 to 17.54 g glutamic acid and glutamine/100g total amino acids;
s. a glycine content of from 2.50 to 6.50 g glycine/100g total amino acids;
t. a proline content of from 1.84 to 5.84 g proline/100g total amino acids;
u. a serine content of from 2.35 to 6.35 g serine/100g total amino acids;
v. a methionine content of from 1.22 to 5.22 g methionine/100g total amino acids;
w. a cysteine and cystine content of from 0.24 to 1.04 g cysteine and cystine/100g total amino acids;
x. a tryptophan content of from 0.76 to 1.76 g tryptophan/100g total amino acids.

In some preferred embodiments, the protein hydrolysate has a fluoride content of from 0.1 to 30 mg/kg on a dry weight basis. In some preferred embodiments, the protein hydrolysate has a TMAO content of from 0.1 to 10 mg N/100 g hydrolysate on a dry weight basis. In some preferred embodiments, the protein hydrolysate has a TMA content of from 0.1 to 30 mg N/100 g hydrolysate on a dry weight basis. In some preferred embodiments, the protein hydrolysate has a TVN content of from 0.1 to 60 mg N/100 g hydrolysate on a dry weight basis.

In some preferred embodiments, the protein hydrolysate further has all of properties g, h, i, j, k, l, m, n, o, p, q, r, s, t, u, v, w and x.

In some preferred embodiments, the essential amino acid content of the hydrolysate is from 39.36 to 49.36 g essential amino acids/100 g total amino acids. In some preferred embodiments, the branched chain amino acid content of the hydrolysate is from 16.35 to 22.35 g branched chain amino acids/100 g total amino acids. In some preferred embodiments, the sulfur-containing amino acid content of the hydrolysate is from 2.86 to 4.86 g sulfur-containing amino acids/100 g total amino acids.

In some preferred embodiments, the protein hydrolysate is further characterized in having a protein content of greater than 89%, 90%, 91%, 92%, 93%, 94% or 95% on a dry weight basis and a fat content of less than 2% on a dry weight basis. In some preferred embodiments, the protein hydrolysate has a moisture content of less than 3%. In some preferred embodiments, the protein hydrolysate is further characterized in having a neutral, non-fishy taste.

In some preferred embodiments, the protein hydrolysate is further characterized in dispersing into a transparent solution when added to water or other aqueous medium. In some preferred embodiments, the OD 590 of a solution of 4.5 g of the protein hydrolysate dry weight in 100 g water is less than 0.4. In some preferred embodiments, the protein hydrolysate is greater than 80% soluble in water as assayed by dissolving 4.5 g of the protein hydrolysate dry weight in 100 g water. In some preferred embodiments, the protein hydrolysate is greater than 60% soluble in water as assayed by dissolving 4.5 g of the protein hydrolysate dry weight in 100 g water and heating to 85°C for five minutes.

In some preferred embodiments, the krill protein hydrolysates have an astaxanthin content of less than 50 ppm.

In some preferred embodiments, the present invention provides processes for producing a krill protein hydrolysate, and in in particularly preferred embodiments krill protein hydrolysates as described above, comprising: providing a krill meal; milling the krill meal; washing the milled krill meal with water and/or aqueous citric acid to provide a washed krill meal; treating the washed krill meal with a protease to provide a krill protein hydrolysate; and filtering the krill hydrolysate by microfiltration with a filter having a pore size of from 0.1 to 10 µm and/or nanofiltration with a filter having a pore size of from 1 to 10 nm to provide a krill protein hydrolysate and a permeate. In some preferred embodiments, the krill meal is a solvent-extracted krill meal. In some preferred embodiments, the processes further comprise the step of deactivating the protease prior to filtration. In some preferred embodiments the processes further comprise the step of drying the krill protein hydrolysate to provide a protein hydrolysate. The krill meal may preferably be a full fat krill meal or a delipidated krill meal. In some preferred embodiments, the protease is non-native as compared to the starting krill meal (i.e., the protease does not naturally occur in the krill used to make the meal).

A composition comprising a krill protein hydrolysate concentrate is also described herein characterized in having a protein content of greater than 85% on a dry weight basis, less than 2% fat on a dry weight basis, and less than 4% moisture.

In some preferred embodiments, the krill protein hydrolysate concentrate is further characterized in having a protein content of greater than 89% on a dry weight basis, less than 1% fat on a dry weight basis, and less than 3% moisture. In some preferred embodiments, the krill protein hydrolysate concentrate is further characterized in having an arginine content of from 5 to 7% (g AA/100 g protein). In some preferred embodiments, the krill protein hydrolysate concentrate is further characterized in having a leucine content of from 8 to 10% (g AA/100 g protein). In some preferred embodiments, the krill protein hydrolysate concentrate is further characterized in having a combined branched chain amino acid (i.e., leucine, isoleucine and valine) content of from 17 to 19.5% (g AA/100 g protein). In some preferred embodiments, the krill protein hydrolysate concentrate is further characterized in having a combined content of methionine and cysteine of from 2 to 4% (g AA/100 g protein). In some preferred embodiments, the krill protein hydrolysate concentrate is further characterized in comprising from 50% to 80% peptides of from 2 to 20 amino acids on a w/w basis (peptides of from 2-20 amino acids on length/total weight of free amino acids peptides and polypeptides).

In some preferred embodiments, the krill protein hydrolysate concentrate is further characterized in comprising less than 60 ppm trimethylamine oxide. In some preferred embodiments, the krill protein hydrolysate concentrate is further characterized in comprising less than 50 ppm fluoride. In some preferred embodiments, the krill protein hydrolysate concentrate is further characterized in comprising less than 1% salt. In some preferred embodiments, the krill protein hydrolysate concentrate is further characterized in having a neutral, non-fishy taste. In some preferred embodiments, the krill protein hydrolysate concentrate is further characterized in dispersing into a transparent or clear solution when added to water or other aqueous medium.

Processes for producing a krill protein hydrolysate concentrate are also described herein comprising: providing a solvent-extracted krill meal, washing the solvent extracted krill meal with water and/or citric acid to provide a washed solvent-extracted krill meal, treating the washed solvent extracted krill meal with a protease to provide a krill protein hydrolysate, and filtering the hydrolysate by microfiltration and/or nanofiltration to provide a krill protein hydrolysate concentrate characterized in comprising from 50% to 80% peptides of from 2 to 10 amino acids on a w/w basis (peptides of from 2-10 amino acids on length/total weight of free amino acids peptides and polypeptides) and a permeate.

In some preferred embodiments, the processes further comprise the step of milling the solvent-extracted krill meal prior to washing. In some preferred embodiments, the processes further comprise the step of deactivating the protease prior to filtration. In some preferred embodiments, the processes further comprise the step of drying the krill protein hydrolysate concentrate to provide a granular krill protein hydrolysate concentrate.

In some preferred embodiments, the granular krill protein hydrolysate is characterized in having a protein content of greater than 85% on a dry weight basis, less than 2% fat on a dry weight basis, and less than 4% moisture. In some preferred embodiments, the granular krill protein hydrolysate concentrate is further characterized in having a protein content of greater than 89% on a dry weight basis, less than 1% fat on a dry weight basis, and less than 3% moisture. In some preferred embodiments, the granular krill protein hydrolysate concentrate is further characterized in having an arginine content of from 5 to 7% (g AA/100 g protein). In some preferred embodiments, the granular krill protein hydrolysate concentrate is further characterized in having a leucine content of from 8 to 10% (g AA/100 g protein). In some preferred embodiments, the granular krill protein hydrolysate concentrate is further characterized in having a combined branched chain amino acid content of from 17 to 19.5% (g AA/100 g protein). In some preferred embodiments, the granular krill protein hydrolysate concentrate is further characterized in having a combined content of methionine and cysteine of from 2 to 4% (g AA/100 g protein).

In some preferred embodiments, the granular krill protein hydrolysate concentrate is further characterized in comprising less than 60 ppm trimethylamine oxide. In some preferred embodiments, the granular krill protein hydrolysate concentrate is further characterized in comprising less than 50 ppm fluoride. In some preferred embodiments, the granular krill protein hydrolysate concentrate is further characterized in comprising less than 1% salt. In some preferred embodiments, the granular krill protein hydrolysate concentrate is further characterized in having a neutral, non-fishy taste. In some preferred embodiments, the granular krill protein hydrolysate concentrate is further characterized in dispersing into a clear solution when added to water or other aqueous medium.

A krill protein hydrolysate concentrate made by the processes described above is also described herein.

### DESCRIPTION OF THE DRAWINGS

FIG. 1 is a bar chart showing the amino acid profile of a krill protein hydrolysate according to the invention.
FIG. 2 is a bar chart showing the peptide size distribution of a krill protein hydrolysate according to the invention.
FIG. 3 is a flow chart of a process according to the invention.

### DEFINITIONS

As used herein, the term "hydrolysate" refers to a mixture of amino acids and peptides of different chain length resulting from the enzymatic hydrolysis of a source protein by an added enzyme such as an exogenous protease or protease that is non-native to the source protein.

As used herein, the term "oral delivery vehicle" refers to any means of delivering a pharmaceutical orally, including, but not limited to, capsules, pills, tablets and syrups.

As used herein, the term "food product" refers to any food or feed suitable for consumption by humans, non-ruminant animals, or ruminant animals. The "food product" may be a prepared and packaged food (e.g., mayonnaise, salad dressing, bread, or cheese food) or an animal feed (e.g., extruded and pelleted animal feed or coarse mixed feed). "Prepared food product" means any pre-packaged food approved for human consumption.

As used herein, the term "foodstuff" refers to any substance fit for human or animal consumption.

As used herein, the term "functional food" refers to a food product to which a biologically active supplement has been added.

As used herein, the term "nutritional supplement" refers to a food product formulated as a dietary or nutritional supplement to be used as part of a diet.

As used herein, the term w/w (weight/weight), unless otherwise specified, refers to the amount of a given substance in a composition on a weight basis and is expressed as a percentage of the total composition weight.

As used herein, the term "krill meal" refers to a powder made from krill. Examples of krill meal include, but are not limited to, dried krill meal (e.g., krill meal with a moisture content of the 3 to 15%), delipidated krill meal (e.g., krill meal that has had fat removed by solvent extraction), full-fat krill meal (krill meal that has not been solvent extracted), and subfractions of krill meal.

As used herein, the term "protein," when used in reference to the protein content of a hydrolysate or composition, refers to the content of polypeptides, peptides and amino acids in the hydrolysate or composition. Unless otherwise noted, the protein content is determined by measuring the total nitrogen content, of, for example a hydrolysate, and multiplying by a conversion factor of 6.25. Any suitable method of determining total nitrogen content may be utilized. For example, in some preferred embodiments, the Kjeldahl method is utilized. The content of amino acids in a hydrolysate may be expressed as grams of the amino acid (e.g., as determined by High Performance Liquid Chromatography (HPLC)) per 100 grams of total protein (e.g., as determined by the Kjeldahl method) or as grams of the amino acid per 100 g of total amino acids (e.g., as determined by HPLC of the 20 common amino acids found in proteins).

As used herein, the term "essential amino acids" refers to the nine amino acids that humans cannot synthesize which are histidine, isoleucine, leucine, lysine, methionine, phenylalanine, threonine, tryptophan, and valine.

As used herein, the term "branched chain amino acids" refers to leucine, isoleucine, and valine.

As used herein the term "sulfur-containing amino acids" refers to methionine and cysteine (which may be expressed as cysteine and cystine in the values reported herein).

### DETAILED DESCRIPTION OF THE INVENTION

The present invention provides krill protein hydrolysates which may be used in pharmaceuticals, nutraceuticals, functional foods, foods, beverages and animal feeds as well as methods for making such protein hydrolysates. In some preferred embodiments, krill protein hydrolysates are prepared from whole krill (e.g., fresh or frozen krill), de-shelled krill, or krill meal. In some particularly preferred embodiments, the krill protein hydrolysates are prepared from krill meal, including but not limited to, dried krill meal, delipidated krill meal, full-fat krill meal and fractions of krill meal. The present invention is not limited to the use of any particular krill species. For example, the krill species may be *Euphausia superba* or *Euphausia pacifica.*

During the development of the invention, it was discovered that it is difficult to process krill meal to provide hydrolysates with an acceptable taste and smell along with properties such as a low fluoride content and unique amino acid profile. Previous krill hydrolysates have been described, for example, in WO2010/030193. While that application describes methods for reducing fluoride, the resulting product (believed to marketed as Rimfrost krill powder), contains high amounts of fat and only approximately 55-60% protein. References such as Zhang et al., Fisheries Sci. (2002) 68:672-679 describe krill hydrolysates but provide no evidence of fluoride or TMAO reduction. The quality of the protein produced in Zhang et al. also appears to be low as evidenced by the reported amino acid composition of the hydrolysate.

The present invention addresses problems associated with taste, smell, and fluoride levels and provides a product with an exceptional amino acid composition by utilizing a multistep process that includes washing and/or milling of the krill meal prior to hydrolysis and subsequent filtering steps including nanofiltration. These compositions and processes are exemplified with krill meal but are equally applicable to other meals. As is shown in the examples, the resulting hydrolysate products are characterized by having improved taste and smell (and associated low levels of TMAO (trimethylamine N-oxide), TMA (trimethylamine) and TVN (Total Volatile Nitrogen)) and low fluoride levels while having a unique amino acid profile. The amino acid profile reveals that the krill protein hydrolysates of the present invention are a complete protein source with sufficient levels of the nine essential amino acids: histidine, isoleucine, leucine, lysine, methionine, phenylalanine, threonine, tryptophan, and valine. As a non-limiting example, the amino acid score for a krill protein hydrolysate of the present invention has been calculated to range from 129-159 in comparison to scores of 121 for egg protein and 115 for whey hydrolysate. Complete proteins have a score of 100 or greater on this scale. The amino acid scores are described in detail in WHO (2007): Protein and amino acid requirements in human nutrition. Thus, the krill protein hydrolysates of the present invention are superior sources of essential amino acids and may be used alone as a complete source of essential amino acids or used to supplement other protein sources which may be lower in essential amino acids.

### 1. Starting materials

In particularly preferred embodiments, the krill protein hydrolysates are prepared from krill meal such as full-fat krill meal or solvent-extracted meal (delipidated krill meal) resulting from extraction of oil from a starting meal, for example, *Euphausia superba* or *Euphausia pacifica* krill meals. Krill meals are preferably made by cooking and drying fresh, most preferably live, krill on board a fishing vessel to provide a krill meal. An exemplary krill meal obtained this process may have an approximate protein content of 70%, approximate fat content of 20% and approximate moisture content of less than 8%.

Krill meal can preferably be made by any standard marine meal process. In general, the krill meal is produced by cooking freshly caught krill at low temperature (approximately 80-85°C) and drying to reduce the moisture content to approximately 5 to 8% and then grinding. In embodiments where the product is intended for human consumption, it is preferable to pack and store the meal under nitrogen without the addition of antioxidants. In some particularly preferred embodiments, the krill meal is a solvent-extracted or delipidated krill meal. The solvent-extracted krill meal may be a residual meal from any type of oil extraction process, for example, extraction with ethanol, methanol, heptane or supercritical solvents such as carbon dioxide with or without an entrainer. In some preferred embodiments, the solvent extracted krill meal is a residual, delipidated krill meal resulting from an extraction process described in PCT/GB2008/001080 or PCT/IB2016/000208.

In some preferred embodiments, krill meal is mixed with a suitable solvent to extract lipids from the meal. An alternative to prior art methods utilizes conditions which preferably extract the maximum amount of lipids from the krill meal at the cost of an increased amount of contaminants in the initial solvent extract. In preferred embodiments, the solvent is an organic protic solvent, however other solvents known for use in extraction of food grade lipids may also be used such as acetone, hexane, etc. Suitable organic protic solvents include, but are not limited to, n-butanol, n-propanol, isopropanol, nitromethane, ethanol, and methanol. In particularly preferred embodiments, the protic solvent is ethanol.

In preferred embodiments, the concentration of the protic solvent used in the initial solvent extraction step is at least 90%, or preferably from about 94% to 98%, more preferably from about 95% to 97%, and is most preferably about 96% (e.g., 96% ethanol or methanol).

In some embodiments, the protic solvent is mixed with the biological starting material at a ratio of protic solvent: biological starting material of about 1:1 to 10:1, preferably about 3:1 to 6:1, more preferably about 4:1 to 5:1, and most preferably about 4.4:1.

In preferred embodiments, the biological starting material is extracted with protic solvent at a temperature of from about 5°C to 65°C, from about 20°C to about 60°C, preferably from about 30°C to 50°C, more preferably from about 30°C to 50°C, and most preferably at about 40°C. In some embodiments, the extraction time (i.e., the amount of time the biological starting material is in contact with the solvent) is from about 10 minutes to about 2 hours, preferably from about 15 minutes to 60 minutes, more preferably from about 20 minutes to about 45 minutes, and most preferably about 30 minutes.

Following the extraction step, a crude krill lipid solution containing the soluble lipids from the krill meal is separated from the solvent/krill meal mixture, for example by decantation and or filtration. The insoluble material, comprising proteins and other useful materials is then dried to recover ethanol. The remaining delipidated krill meal may then be used to make a hydrolysate according to the present invention. In some preferred embodiments, the delipidated krill meal contains less than 15% or 12% wt/wt fat, greater than 65% or 70% wt/wt crude protein and/or less than 5% or 3% w/w moisture.

### 2. Hydrolysate process and compositions

In some preferred embodiments, the present invention provides processes for producing a krill protein hydrolysate comprising providing a krill meal, milling the krill meal, washing the krill meal with water and/or citric acid to provide a washed krill meal, and treating the krill meal with a protease to provide a hydrolysate, and filtering the hydrolysate by microfiltration and/or nanofiltration. In some particularly preferred embodiments, the krill meal is a delipidated krill meal or full fat krill meal. In some preferred embodiments, where the meal is a solvent-extracted krill meal, the hydrolysate obtained after filtration is characterized in in comprising from 50% to 80% peptides of from 2 to 10 amino acids on a w/w basis (peptides of from 2-10 amino acids on length/total weight of free amino acids peptides and polypeptides) and a permeate. Additional characteristics of preferred krill protein hydrolysates produced by the process are described in detail below.

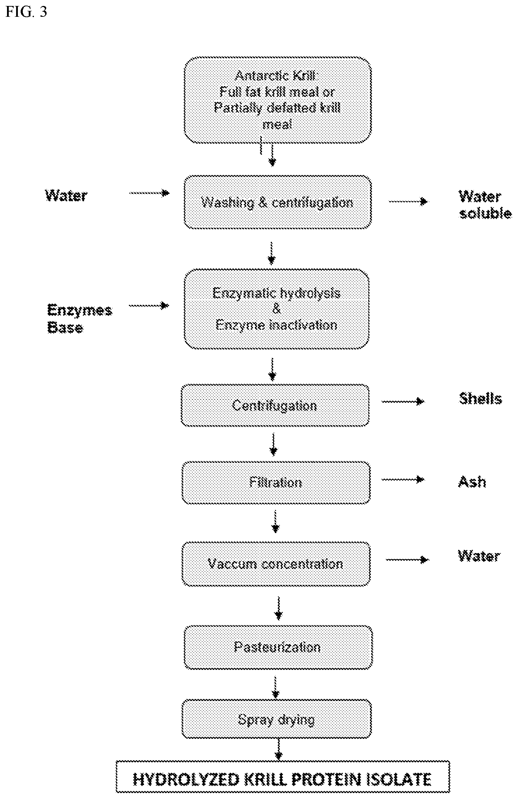

In some preferred embodiments, the processes comprise one or more additional steps, such as those depicted in FIG. 3. The krill meal is milled, preferably wet-milled, prior to washing to reduce particle size. In some preferred embodiments, the milling step reduces particle size of the meal to less than 100 µm and preferably to about 50 µm. The meal is then washed with water and/or citric acid. In some embodiments, the washing step comprises a combination of water and acidic washing steps. For example, in some embodiments, the solvent-extracted meal is washed first with several volumes of water, followed by a citric acid wash, and then one or more water rinses.

The present invention is not limited to the use of any particular protease or peptidase. In some preferred embodiments, the protease is a serine protease. Suitable serine proteases include subtilisinA and other subtilisinS obtained from, for example, *Bacillus subtilis.* Suitable commercial proteases are marketed by Novozymes and include, but are not limited to, ALCALASE^{™} 2.4 L FG, ALCALASE^{™} 2.5 L, SAVINASE^{™} 12 T, SAVINASE^{™} 16 L, and ESPERASE^{™} 8.0 L. In some preferred embodiments, the processes comprise treating washed and milled solvent-extracted krill meal with ALCALASE^{™} at pH of 5 to 9, preferably 7 to 8, and most preferably 7.5 at a temperature of from 30 to 70 °C, more preferably 50 to 60°C, and most preferably at about 60°C, for up to 24 hours (e.g., 30 minutes to 24 hours), for instance from 1-3 hours, about 2 hours, or about 1 hour. Other suitable proteases include, but are not limited to, cysteine proteases, threonine proteases, aspartic proteases, glutamic proteases, metalloproteases and asparagine peptide lysases as are known in the art.

In some preferred embodiments, the processes comprise the step of deactivating the protease prior to filtration. In further preferred embodiments, the processes comprise the step of drying the protein hydrolysate to provide a granular protein hydrolysate. The drying step may be performed by methods known in the art including spray drying. As described in Fig. 3, the process may also preferably comprise additional steps of vacuum concentration, pasteurization and filtration. Nanofiltration generally utilizes filters, such as membrane filters, with a pore size of from about 1 to 10 nanometers and, for example, a molecular weight cutoff of approximately 300 Da. A diafiltration step may be utilized in addition to the nanofiltration step.

The processes of the present invention produce krill protein hydrolysates with improved organoleptic properties, including improved taste and smell (as evidenced by low TMA, TMAO and TVN levels) as well as low fluoride levels compared to the starting raw material. In some preferred embodiments, the krill protein hydrolysates are characterized in having a protein content of greater than 85% on a dry weight basis, less than 2% fat on a dry weight basis, and less than 4% moisture. In some preferred embodiments, the krill protein hydrolysate is further characterized in having a protein content of greater than 89% on a dry weight basis, less than 1% fat on a dry weight basis, and less than 3% moisture.

In some particularly preferred embodiments, krill hydrolysates are characterized in having a protein content of greater than 85% on a dry weight basis, less than 5% fat on a dry weight basis, and property a to f.
a. a fluoride content of from 0.1 to 200 mg/kg on a dry weight basis, preferably from 0.1 to 30 mg/kg on a dry weight basis; more preferably from 0.1 to 10 mg/kg; even more preferably from 0.1 to 5 mg/kg on a dry weight basis; and most preferably from 0.1 to 3 mg/kg on a dry weight basis; or alternatively, less than 200, 30, 10, 3 or 1 mg/kg F on a dry weight basis;
b. a TMAO content of from 0.1 to 200 mg N/100g hydrolysate on a dry weight basis, preferably from 0.1 to 100 mg N/100g hydrolysate on a dry weight basis; more preferably from 0.1 to 30 mg N/100g hydrolysate on a dry weight basis; even more preferably from 0.1 to 25 mg N/100g hydrolysate on a dry weight basis; and most preferably from 0.1 to 10 mg N/100g hydrolysate on a dry weight basis;
c. a TMA content of from 0.1 to 200 mg N/100g hydrolysate on a dry weight basis, preferably from 0.1 to 100 mg N/100g hydrolysate on a dry weight basis; more preferably from 0.1 to 50 mg N/100g hydrolysate on a dry weight basis; even more preferably from 0.1 to 30 mg N/100g hydrolysate on a dry weight basis; and most preferably from 0.1 to 10 mg N/100g hydrolysate on a dry weight basis;
d. a TVN content of from 0.1 to 200 mg N/100g hydrolysate on a dry weight basis, preferably from 0.1 to 100 mg N/100g hydrolysate on a dry weight basis; more preferably from 0.1 to 60 mg N/100g hydrolysate on a dry weight basis; and most preferably from 0.1 to 20 mg N/100g hydrolysate on a dry weight basis;
e. a sodium content of from 0.01 to 4.0 g/100 g hydrolysate on a dry weight basis, more preferably from 0.1 to 1.0 g/100g hydrolysate on a dry weight basis; and most preferably from 0.01 to 0.2 g/100g hydrolysate on a dry weight basis;
f. a calcium content of from 100 to 25,000 mg/kg hydrolysate on a dry weight basis, more preferably from 10,000 to 25,000 mg/kg hydrolysate on a dry weight basis; and most preferably from 12,000 to 16,000 mg/kg hydrolysate on a dry weight basis.

The krill protein hydrolysate of the present invention may also have one or more of the following properties:
g. a lysine content of from 6.30 to 10.30 g lysine/100 g total amino acids, preferably from 7.30 to 9.30 g lysine/100 g total amino acids, and more preferably from 7.80 to 8.80 g lysine/100 g total amino acids or alternatively from 6.29 to 10.29 g lysine/100g protein, preferably from 7.29 to 9.29 g lysine/100g protein, and more preferably from 7.79 to 8.79 g lysine/100g protein;
h. a threonine content of from 3.04 to 7.04 grams threonine/100 g total amino acids, preferably from 4.04 to 6.04 g threonine/100 g total amino acids or more preferably from 4.54 to 5.54 g threonine/100 g total amino acids, or alternatively from 3.36 to 7.36 g threonine/100g protein, preferably from 4.36 to 6.36 g threonine/100g protein, more preferably from 4.86 to 5.86 g threonine/100g protein;
i. an isoleucine content of from 3.39 to 7.39 g isoleucine/100 g total amino acids, preferably from 4.39 to 6.39 g isoleucine/100 g total amino acids and more preferably from 4.89 to 5.89 g isoleucine/100 g total amino acids, or alternatively from 3.38 to 7.38 g isoleucine/100g protein, preferably from 4.38 to 6.38 g isoleucine/100g protein, more preferably from 4.88 to 5.88 g isoleucine/100g protein;
j. a leucine content of from 6.27 to 10.27 g leucine/100 g total amino acids, preferably from 7.27 to 9.27 g leucine/100 g total amino acids, or more preferably from 7.77 to 8.77 g leucine/100 g total amino acids, or alternatively from 6.37 to 10.37 g leucine/100g protein, preferably from 7.37 to 9.37 g leucine/100g protein, more preferably from 7.87 to 8.87 g leucine/100g protein;
k. a histidine content of from 0.94 to 3.94 g histidine/100 g total amino acids, preferably from 1.44 to 3.44 g histidine/100 g total amino acids, and more preferably from 1.94 to 2.94 g histidine/100 g total amino acids, or alternatively from 1.09 to 4.09 g histidine/100g protein, preferably from 1.59 to 3.69 g histidine/100g protein, more preferably from 2.09 to 3.09 g histidine/100g protein;
l. a phenylalanine content of from 2.76 to 6.76 g phenylalanine/100 g total amino acids, preferably from 3.76 to 5.76 g phenylalanine/100 g total amino acids, and more preferably from 4.26 to 5.26 g phenylalanine/100 g total amino acids, or alternatively from 3.05 to 7.05 g phenylalanine/100g protein, preferably from 4.05 to 6.05 g phenylalanine/100g protein, more preferably from 4.55 to 5.55 g phenylalanine/100g protein;
m. a tyrosine content of from 2.39 to 6.39 g tyrosine/100 g total amino acids, preferably from 3.39 to 5.39 g tyrosine/100 g total amino acids, and more preferably from 3.89 to 4.89 g tyrosine/100 g total amino acids, or alternatively from 2.46 to 6.46 g tyrosine/100g protein, preferably from 3.46 to 5.46 g tyrosine/100g protein, more preferably from 3.96 to 4.96 g tyrosine/100g protein;
n. a valine content of from 3.69 to 7.69 g valine/100 g total amino acids, preferably from 4.69 to 6.69 g valine/100 g total amino acids, and more preferably from 5.19 to 6.19 g valine/100g total amino acids, or alternatively from 3.88 to 7.88 g valine/100g protein, preferably from 4.88 to 6.88 g valine/100g protein, more preferably from 5.38 to 6.38 g valine/100g protein;
o. an alanine content of from 3.76 to 7.76 g alanine/100 g total amino acids, preferably from 4.76 to 6.76 g alanine/100 g total amino acids, or more preferably from 5.26 to 6.26 g alanine/100 g total amino acids, or alternatively from 3.80 to 7.80 g alanine/100g protein, preferably from 4.80 to 6.80 g alanine/100g protein, more preferably from 5.30 to 6.30 g alanine/100g protein;
p. an arginine content of from 3.90 to 7.90 g arginine/100 g total amino acids, preferably from 4.90 to 6.90 g arginine/100 g total amino acids, or more preferably from 5.40 to 6.40 g arginine/100 g total amino acids, or alternatively from 3.98 to 7.98 g arginine/100g protein, preferably from 4.98 to 6.98 g arginine/100g protein, more preferably from 5.48 to 6.48 g arginine/100g protein;
q. an aspartic acid/asparagine content of from 9.68 to 13.68 g aspartic acid and asparagine/100 g total amino acids, preferably from 10.68 to 12.68 g aspartic acid and asparagine /100 g total amino acids, or more preferably from 11.18 to 12.18 g aspartic acid and asparagine /100 g total amino acids, or alternatively from 10.37 to 14.37 g aspartic acid and asparagine /100g protein, preferably from 11.37 to 13.37 g aspartic acid and asparagine /100g protein, more preferably from 11.87 to 12.87 g aspartic acid and asparagine /100g protein;
r. a glutamic acid/glutamine content of from 11.54 to 17.54 g glutamic acid and glutamine/100 g total amino acids, preferably from 12.54 to 16.54 g glutamic acid and glutamine /100 g total amino acids, or more preferably from 13.54 to 15.54 g glutamic acid and glutamine /100 g total amino acids, or alternatively from 11.66 to 17.66 g glutamic acid and glutamine /100g protein, preferably from 12.66 to 16.66 g glutamic acid and glutamine /100g protein, more preferably from 13.66 to 15.66 g glutamic acid and glutamine /100g protein;
s. a glycine content of from 2.50 to 6.50 glycine/100 g total amino acids, preferably from 3.50 to 5.50 g glycine/100 g total amino acids, and more preferably from 4.00 to 5.00 g glycine/100 g total amino acids or alternatively from 2.70 to 6.70 g glycine/100g protein, preferably from 3.70 to 5.70 g glycine/100g protein, more preferably from 4.20 to 5.20 g glycine/100g protein;
t. a proline content of from 1.84 to 5.84 g proline/100 g total amino acids, preferably from 2.84 to 4.84 g proline/100 g total amino acids, or more preferably from 3.34 to 4.34 g proline/100 g total amino acids, or alternatively from 2.27 to 6.27 g proline/100g protein, preferably from 3.27 to 5.27 g proline/100g protein, more preferably from 3.77 to 4.77 g proline/100g protein;
u. a serine content of from 2.35 to 6.35 g serine/100 a total amino acid, preferably from 3.35 to 5.35 g serine/100 g total amino acids, and more preferably from 3.85 to 4.85 g serine/100 g total amino acids, or alternatively from 2.64 to 6.64 g serine/100g protein, preferably from 3.64 to 5.64 g serine/100g protein, more preferably from 4.14 to 5.14 g serine/100g protein;
v. a methionine content of from 1.22 to 5.22 g methionine/100 g total amino acids, preferably from 2.22 to 4.22 g methionine/100 g total amino acids, or more preferably 2.72 to 3.72 g methionine/100 g total amino acids, or alternatively from 1.69 to 5.69 g methionine/100g protein, preferably from 2.69 to 4.69 g methionine/100g protein, more preferably from 3.19 to 4.19 g methionine/100g protein;
w. a cysteine/cystine content of from 0.24 to 1.04 g cysteine and cystine/100 g total amino acids, preferably from 0.44 to 0.84 g cysteine and cystine /100 g total amino acids, or more preferably from 0.54 to 0.74 cysteine and cystine/100 g total amino acids, or alternatively from 0.249 to 1.049 g cysteine and cystine/100g protein, preferably from 0.449 to 0.849 g cysteine and cystine/100g protein, more preferably from 0.549 to 0.749 g cysteine and cystine/100g protein;
x. a tryptophan content of from 0.76 to 1.76 g tryptophan/100 g total amino acids, preferably from 0.96 to 1.56 g tryptophan/100 g total amino acids, or more preferably from 1.06 to 1.46 g tryptophan/100 g total amino acids, or alternatively from 0.8 to 1.8 g tryptophan/100g protein, preferably from 1.0 to 1.6 g tryptophan/100g protein, and, more preferably from 1.1 to 1.5 g tryptophan/100g protein.

As can be seen, the ranges for total amino acids content are provided in grams of the specified amino acid per 100 grams of total amino acids (of the 20 amino acids found in proteins) as measured by HPLC or alternatively as grams of amino acid (measured by HPLC) per 100 grams of total protein (e.g., as measured by the Kjeldahl method).

In some even more preferred embodiments, the hydrolysates described above have a protein content of greater than 90%, 91%, 92%, 93%, 94% or 95% on a dry weight basis and/or a fat content of less than 3%, 2% or 1% on a dry weight basis.

In some preferred embodiments, the hydrolysates further have a fluoride content of from 0.1 to 30 ppm, and/or a TMAO content of from 0.1 to 10 ppm; and/or a TMA content of from 0.1 to 30 ppm; and/or a TVN content of from 0.1 to 60 ppm.

In some preferred embodiments, the hydrolysates further have all of properties g, h, i, j, k, l, m, n, o, p, q, r, s, t, u, v, w, and x in addition to all of properties a, b, c, d, e and f.

In some preferred embodiments, the hydrolysates are further characterized in having an essential amino acid content of from 39.36 to 49.36 g essential amino acids/100 g total amino acids, preferably from 41.36 to 47.36 g essential amino acids/100 g total amino acids, more preferably from 42.36 to 46.36 g essential amino acids/100 g total amino acids, and most preferably from 43.36 to 45.36 g essential amino acids/100 g total amino acids.

In some preferred embodiments, the hydrolysates are further characterized in having a branched chain amino acid content of from 16.35 to 22.35 g branched chain amino acids/100 g total amino acids, 17.35 to 21.35 g branched chain amino acids/100 g total amino acids, and more preferably from 18.35 to 20.35 g branched chain amino acids/100 g total amino acids.

In some preferred embodiments, the hydrolysates are further characterized in have a sulfur-containing amino acid content of from 2.86 to 4.86 g sulfur-containing amino acids/100 g total amino acids, preferably from 3.16 to 4.56 g sulfur-containing amino acids/100 g total amino acids, and more preferably from 3.46 to 4.26 g sulfur-containing amino acids/100 g total amino acids.

In some preferred embodiments, the protein hydrolysates are further characterized in having an arginine content of from 5 to 7% (g Amino Acid ("AA")/100 g protein). In some preferred embodiments, the protein hydrolysates are further characterized in having a leucine content of from 7 to 10% (g AA/100 g protein). In some preferred embodiments, the protein hydrolysates are further characterized in having a combined branched chain amino acid content of from 17 to 19.5% (g AA/100 g protein). In some preferred embodiments, the protein hydrolysates are further characterized in having a combined content of methionine and cysteine of from 2 to 4% (g AA/100 g protein). In some preferred embodiments, the protein hydrolysates are further characterized in having a content of tryptophan of from 0.1% to 3% (g AA/100 g protein), more preferably from 0.2% to 2% (g AA/100 g protein). In some preferred embodiments, the protein hydrolysates are further characterized in comprising from 50% to 80% peptides of from 2 to 10 amino acids on a w/w basis (peptides of from 2-10 amino acids on length/total weight of free amino acids peptides and polypeptides). In some preferred embodiments, the protein hydrolysates are further characterized in comprising from 50% to 80% peptides of from 2 to 20 amino acids on a w/w basis (peptides of from 2-20 amino acids on length/total weight of free amino acids peptides and polypeptides). In some preferred embodiments, the protein hydrolysates are further characterized in comprising from 50% to 80% peptides of from 2 to 15 amino acids on a w/w basis (peptides of from 2-15 amino acids on length/total weight of free amino acids peptides and polypeptides). In some preferred embodiments, the protein hydrolysates are is further characterized in comprising from 50% to 80% peptides of from 2 to 20 amino acids on a w/w basis (peptides of from 2-10 amino acids on length/total weight of free amino acids peptides and polypeptides).

In some preferred embodiments, the krill protein hydrolysates are further characterized in comprising less than 60 ppm trimethylamine oxide. In some preferred embodiments, the krill protein hydrolysates are further characterized in comprising less than 50 ppm fluoride. In some preferred embodiments, the krill protein hydrolysates are further characterized in comprising less than 4% and most preferably less than 1% sodium. In some preferred embodiments, the krill protein hydrolysates are further characterized in having a neutral, non-fishy taste.

In some preferred embodiments, the krill protein hydrolysates are further characterized in dispersing into a clear solution when added to water or other aqueous medium. For example, in some embodiments, when 4.5 grams of a hydrolysate of the present invention on a dry weight basis is dissolved into 100 g water at pH 3-9 (e.g., pH 6) the optical density at 590 nm (OD 590) is less than 0.4, preferably less than 0.3. In some embodiments, the hydrolysates preferably have from 0.1 to 50 ppm, 0.1 to 10 ppm or 0.1 to 5 ppm astaxanthin and more preferably less than 50 ppm, 10 ppm or 5ppm astaxanthin.

In some embodiments, the solubility of the krill protein hydrolysates may be assayed by dissolving 4.5 gram of the hydrolysate on a dry weight basis in 100 grams of water at 20 - 25°C (e.g., 23°C), stirring, centrifuging the solution at 1500 g for 5 minutes, and measuring the dry weight of the material in solution after centrifugation. In some preferred embodiments, the solubility of the hydrolysate is more than 80%, 90%, 91%, 92%, 92%, 94% at pH 3-7 (e.g., pH 3.5) as measured by this assay and more than 75%, 80% or 90% at pH 9. In some embodiments, solubility following heat treatment at 85°C for 5 minutes is greater than 60% as measured by the assay at pH 3-9 (e.g., pH 6).

### 3. Hydrolysate products

The hydrolysates of the present invention may be incorporated into a variety of products. Accordingly, foods, beverages, functional foods, animal feed rations, powdered protein supplements, meal replacements, beverage powders, protein shake powders and the like comprising a krill protein hydrolysate accordingly to the invention are described herein.

The hydrolysates of the present invention may be administered orally. Accordingly, the hydrolysates of this invention (such as those described in the preceding sections) may be formulated with acceptable excipients and/or carriers for oral consumption. The actual form of the carrier, and thus, the composition itself, is not critical. The carrier may be a liquid, gel, gelcap, capsule, powder, solid tablet (coated or non-coated), tea, or the like. Oral delivery vehicles are preferably in the form of a tablet or capsule. Suitable excipient and/or carriers include vegetable oil, fish oil, krill oil, maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof). Preferred carriers include calcium carbonate, magnesium stearate, maltodextrin, and mixtures thereof. The various ingredients and the excipient and/or carrier are mixed and formed into the desired form using conventional techniques. The tablet or capsule may be coated with an enteric coating that dissolves at a pH of about 6.0 to 7.0. A suitable enteric coating that dissolves in the small intestine but not in the stomach is cellulose acetate phthalate. Further details on techniques for formulation for and administration may be found in the latest edition of Remington's Pharmaceutical Sciences (Maack Publishing Co., Easton, PA).

The hydrolysates may be formulated for oral administration with flavoring agents or sweeteners. Examples of useful flavoring include, but are not limited to, pure anise extract, imitation banana extract, imitation cherry extract, chocolate extract, pure lemon extract, pure orange extract, pure peppermint extract, imitation pineapple extract, imitation rum extract, imitation strawberry extract, or pure vanilla extract; or volatile oils, such as balm oil, bay oil, bergamot oil, cedarwood oil, walnut oil, cherry oil, cinnamon oil, clove oil, or peppermint oil; peanut butter, chocolate flavoring, vanilla cookie crumb, butterscotch or toffee. In one embodiment, the dietary supplement contains cocoa or chocolate. Emulsifiers may be added for stability of the final product. Examples of suitable emulsifiers include, but are not limited to, lecithin (e.g., from egg or soy), and/or mono- and di-glycerides. Other emulsifiers are readily apparent to the skilled artisan and selection of suitable emulsifier(s) will depend, in part, upon the formulation and final product. In addition to the carbohydrates described above, the nutritional supplement can contain natural or artificial (preferably low calorie) sweeteners, e.g., saccharides, cyclamates, aspartamine, aspartame, acesulfame K, and/or sorbitol.

The dietary supplement may comprise one or more inert ingredients, especially if it is desirable to limit the number of calories added to the diet by the dietary supplement. For example, the dietary supplement may also contain optional ingredients including, for example, herbs, vitamins, minerals, enhancers, colorants, sweeteners, flavorants, inert ingredients, and the like. For example, the dietary supplement may contain one or more of the following: ascorbates (ascorbic acid, mineral ascorbate salts, rose hips, acerola, and the like), dehydroepiandosterone (DHEA), green tea (polyphenols), inositol, kelp, dulse, flavonoids, maltodextrin, nettles, niacin, niacinamide, rosemary, selenium, silica (silicon dioxide, silica gel, horsetail, shavegrass, and the like), spirulina, zinc, and the like. Such optional ingredients may be either naturally occurring or concentrated forms.

The dietary supplements may further comprise vitamins and minerals including, but not limited to, calcium phosphate or acetate, tribasic; potassium phosphate, dibasic; magnesium sulfate or oxide; salt (sodium chloride); potassium chloride or acetate; ascorbic acid; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D₃; cyanocobalamin; sodium selenite; copper sulfate; vitamin A; vitamin C; inositol; potassium iodide. Suitable dosages for vitamins and minerals may be obtained, for example, by consulting the U.S. RDA guidelines.

Nutritional supplements (e.g., energy bars or meal replacement bars or beverages) comprising the hydrolysates of the present invention are also described herein. The nutritional supplement may serve as meal or snack replacement and generally provide nutrient calories. Preferably, the nutritional supplements provide carbohydrates, proteins, and fats in balanced amounts. The nutritional supplement can further comprise carbohydrate, simple, medium chain length, or polysaccharides, or a combination thereof. A simple sugar can be chosen for desirable organoleptic properties. Uncooked cornstarch is one example of a complex carbohydrate. If it is desired that it should maintain its high molecular weight structure, it should be included only in food formulations or portions thereof which are not cooked or heat processed since the heat will break down the complex carbohydrate into simple carbohydrates, wherein simple carbohydrates are mono- or disaccharides. The nutritional supplement contains, in one embodiment, combinations of sources of carbohydrate of three levels of chain length (simple, medium and complex; e.g., sucrose, maltodextrins, and uncooked cornstarch).

Food products, prepared food products, or other foodstuffs (e.g., functional foods) comprising the hydrolysates of the present invention are also described herein. In preferred embodiments, the foods comprise an effective amount of the components as described above. For example, in some embodiments, beverages and solid or semi-solid foods comprising the hydrolysates are provided. These forms can include, but are not limited to, beverages (e.g., soft drinks, milk and other dairy drinks, and diet drinks), baked goods, puddings, dairy products, confections, snack foods, or frozen confections or novelties (e.g., ice cream, milk shakes), prepared frozen meals, candy, snack products (e.g., chips), soups, spreads, sauces, salad dressings, prepared meat products, cheese, yogurt and any other fat or oil containing foods, and food ingredients (e.g., wheat flour).

The hydrolysates may be incorporated into chewable matrices. Preferred chewable matrices jelly candies and gelatin-based gummi candy. Exemplary gummi candies include gummi bears, gummi worms, gummi frogs, gummi hamburgers, gummi cherries, gummi soda bottles, gummi sharks, gummi army men, gummi hippopotami, gummi lobsters, gummi watermelons, gummi octopuses, gummi apples, gummi peaches, and gummi oranges. The terms "gummi" and "gummy" are used interchangeably herein.

As will be appreciated, the hydrolysates of the present invention may be delivered as dietary supplements, nutritional supplements, or functional foods or be incorporated into foods or beverages. Accordingly, methods for preparing a dietary supplement, nutritional supplement, functional food, food or beverage comprising mixing a protein hydrolysate as described above with one or more additional components to form the desired product (i.e., dietary supplement, nutritional supplement, functional food, food or beverage) are also described herein. The additional components for these products are described in detail above. Further, the protein hydrolysates described herein may be used as in ingredient in the preparation of a dietary supplement, nutritional supplement, functional food, food or beverage.

The hydrolysates may be incorporated into animal feeds and rations. Animals feeds may be formulated for companion animals such as cats and dogs as well as domestic and wild animals including domestic poultry, hogs, cattle, sheep, rabbits, and birds, fish such as trout, salmon, catfish and tilapia, shrimp, and other species produced via aquaculture. Many different feed rations may be formulated for animals from many different feed ingredients. Rations are generally formulated to provide nutrients in accordance with National Research Council standards. The feedstuffs used in the ration (in addition to the hydrolysates) can be chosen according to market price and availability. Thus, some components of the ration may change over time. In the feeds, the ration will contain a hydrolysate according to the invention as part of the protein component, but other components may vary over time based on the price of the component. For discussions on feed ration formulation, actual rations and NRC guidelines, see Church, Livestock Feeds and Feeding, O&B Books, Inc., Corvallis, Oreg. (1984) and Feeds and Nutrition Digest, Ensminger, Oldfield and Heineman eds., Ensminger Publishing Corporation, Clovis, Calif. (1990).

The animal feed rations may be characterized according to NRC requirements. NRC requirements may be found in Church, Livestock Feeds and Feeding, O&B Books, Inc., Corvallis, Oreg. (1984), or other nutritional standards. Animal rations are traditionally balanced using the protein and energy requirements, and then adjusted if needed to meet the other requirements. The animal rations will contain a hydrolysate according to the present invention, for example from 0.5% to 30% wt/wt, 0.5% to 20% wt/wt. 0.5% to 10% wt/wt or 0.5% to 5% wt/wt hydrolysate in addition to other feed materials necessary to balance the feed to meet the NRC requirements for the different stages of growth and maintenance. The relative amounts of protein and energy are adjusted to reflect Nutritional Standards requirements. The amounts of feed components will vary with the stage of animal fed. A growing ration for young animals will have higher protein levels, while a finishing ration for finishing animals for market will have higher energy values which are supplied by carbohydrates. In some feeding situations, care must be taken to provide the appropriate amino acids as well as overall protein content. In most animal diets, energy requirements are met by starches in cereal grains. Energy requirements may also be met by addition of fat to the ration. The CFAP provides part of the energy requirement.

Other ingredients may be added to the feed ration. These ingredients include, but are not limited to, mineral supplements such as calcium, phosphorus, salt, selenium and zinc; vitamin supplements such as Vitamins A, B, D, E, and K; amino acid supplements such as lysine; coccidiostats, or growth promoters such as bacitracin or virginamycin; and other active drugs such as chlortetracycline, sulfathiozole, and penicillin. For vitamin, mineral and antibiotic supplement formulation see Church, Livestock Feeds and Feeding, O&B Books, Inc., Corvallis, Oreg. (1984).

The hydrolysate may be incorporated into a pelleted feed for administration to domestic animals. Pelleted feed is created by first mixing feed components and then compacting and extruding the feed components through a die with heat and pressure. The feed is pelleted by methods known in the art, which are described in MacBain, Pelleting Animal Feed, American Feed Manufacturers Association, Arlington, Va. (1974). When incorporating added fat into pelleted feed, caution is needed in order to avoid making mealy pellets. Generally, only about 2% of the fat is added during pelleting, with the rest added after the pellets have cooled.

### Example 1

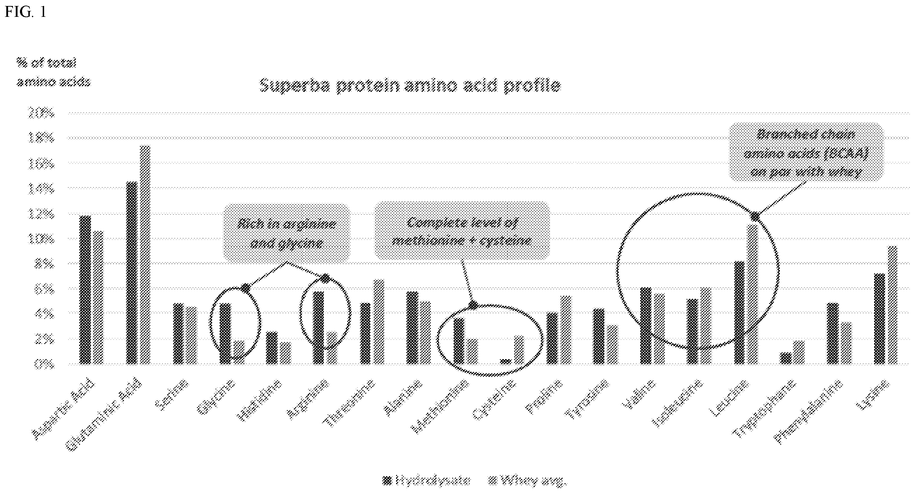

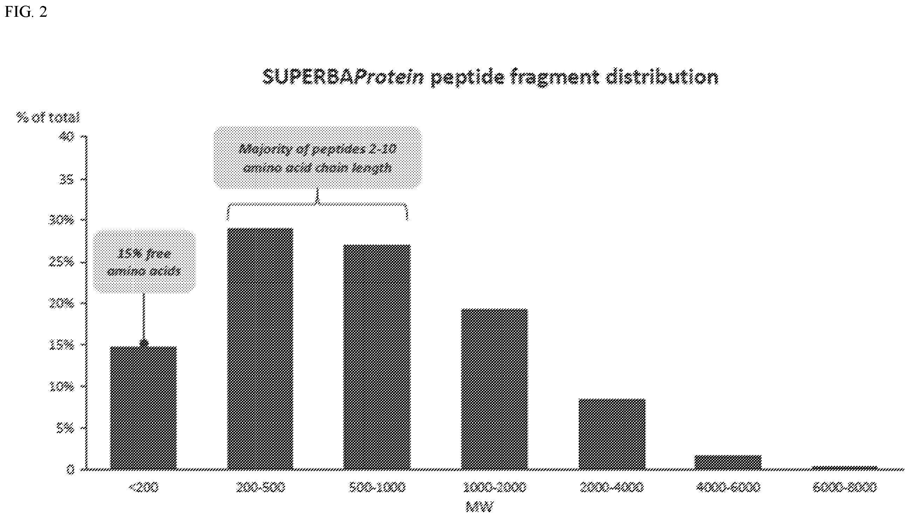

Solvent-extracted krill meal produced according to the process described in PCT/IB2016/000208 is milled in a pin meal at 16,000 RPM to a mean particle size of 50 µm. The milled protein composition is then washed with citric acid (65°C, 9 min., 0.19M, pH 4) followed by a 3 hot water washes at 65°C for 9 min. The washed protein composition is then treated with ALCALASE for 2.5 hours at pH 7.5 and 52.5°C and then washed with hot water for 9 min. at 65°C. The resulting hydrolysate is then micro- and nanofiltered (1.25 L/h/m2/bar, < 5 bar pressure) to provide a concentrate. The concentrate is then granulated by spray drying (agglomeration, three stage drying, inlet 182°C, outlet 82.5°C). The hydrolysate concentrate has the properties listed in Tables 1-3 and in Figs. 1 and 2.

**Table 1.**

| **Parameter:** | **Limits:** | **Method of analysis:** |
|---|---|---|
| **Physical properties** | | |
| Flavor/Odor | Bland | Organoleptic |
| Appearance | Off-white powder | Visual |

| **Chemical analysis** | | |
|---|---|---|
| Moisture | Max 3.0% | Drying |
| Protein, As is | Min 87.5 % | Kjeldahl |
| Protein, Dry basis | Min 90.0 % | Kjeldahl |
| Total Fat | Max 1.0% | Soxhlet |
| Ash | Max 6.0 % | Gravimetric |
| Carbohydrates | Max 5.0% | HPLC |
| Sodium (Na) | Max 1.0% | ICP-MS |

| **Microbiology** | | |
|---|---|---|
| Total Plate Count | Max 1,000 CFU/g | USP |
| Salmonella | Negative / 25 g | USP |
| Enterobacteriacea | Negative / 25 g | USP |
| E. coli | Negative / 10 g | USP |
| Yeast and Mold | Max 100 CFU/g | USP |

**Table 2.**

| **Typical Quantity per 100 g Product** | | |
|---|---|---|
| **Calories (kcal)** | | 373 |
| | - From total fat | 9 |
| | - From Carbohydrate | 8 |
| | - From Protein | 356 |
| | | |
| **Protein** | | **89 g** |
| **Moisture** | | **2g** |
| **Ash** | | **4g** |
| **Crude Fat** | | **1 g** |
| **Total Carbohydrate** | | **2g** |

**Table 3.**

| **Amino Acid content** | **Typical g AA/100g product** | **Typical g AA/100g protein** |
|---|---|---|
| Alanine | 4.0 | 4.3 |
| Arginine | 5.4 | 5.8 |
| Aspartic Acid | 7.6 | 8.2 |
| Cysteine | 0.4 | 0.5 |
| Glutamic Acid | 9.8 | 10.6 |
| Glycine | 5.3 | 5.7 |
| Histidine | 2.8 | 3.1 |
| Isoleucine | 4.1 | 4.5 |
| Leucine | 8.5 | 9.3 |
| Lysine | 5.9 | 6.4 |
| Methionine | 2.4 | 2.6 |
| Phenylalanine | 5.8 | 6.3 |
| Proline | 2.7 | 3.0 |
| Serine | 3.2 | 3.4 |
| Threonine | 3.6 | 3.9 |
| Tyrosine | 3.3 | 3.5 |
| Tryptophan | Not determined | Not determined |
| Valine | 4.2 | 4.6 |

### Example 2

Two processes for making a krill hydrolysate were compared. Except as noted, the process in Fig. 3 was utilized. In the first process, there was no wet milling and no ultrafiltration step. The hydrolysis time was 1 hour. The resulting hydrolysate is referred to as "Batch A." A nanofiltration step was utilized after hydrolysis. The second process utilized wet milling prior to hydrolysis and both ultrafiltration and nanofiltration after hydrolysis. The hydrolysis time was 14.5 hours. The resulting hydrolysate is referred to as "Batch B." A comparison of selected values for Batch A and Batch B is provided in Table 4. Table 5 provides the amino acid composition of Batch B and also average amino acid content across multiple krill hydrolysate batches made by the process of the invention. In the third column of Table 5, the amino acid content is expressed as grams amino acid per 100 grams protein. The grams of amino acid in 100 g hydrolysate was determined by high performance liquid chromatography (HPLC). The protein content was determined by the Kjeldahl method. In the fourth column of Table 5, amino acid content is listed for combined batches made from different meals, including delipidated and full fat krill meals. In column 4, the amino acid content is listed in grams of the indicated amino acid per 100 grams total amino acids (as measured by HPLC). As can be seen, the Batch B hydrolysate has substantially improved values for fluoride (F) content, TMAO, TMA and TVN content. A subsequent batch ("Batch C") was found to have a F content of 1.09 mg/kg on a dry weight basis as well as low values for TMA, TMAO, and TVN. The hydrolysates have excellent amino acid composition, especially with respect to the content and composition of the essential and branched chain amino acids.

**Table 4. Comparison of Batch A hydrolysate and Batch B hydrolysate.**

| **Property** | **BATCH A** | **BATCH B** |
|---|---|---|
| F (mg/kg dry weight basis) | 277 | 8.39 |
| TMAO (mg N/100g, dry weight basis) | 60 | 3 |
| TMA (mg N/100g, dry weight basis) | | <30 |
| TVN (mg N/100g, dry weight basis) | | 37.7 |
| Protein% as is | 87.3 | 92.1 |
| Fat% as is | 0.175 | <0.4 |
| Water content% as is | 4.6 | 1.4 |
| Water activity | | 0.14 |

**Table 5. Amino acid composition of Batch B hydrolysate and average amino acid content across batches.**

| | **Batch B** | | **Average across batches** |
|---|---|---|---|
| **Amino Acid** | **g/100g hydrolysate** | **g/100g protein** | **g /100 g total amino acids** |
| Lysine | 7.64 | 8.29 | 8.30 |
| Threonine | 4.94 | 5.36 | 5.04 |
| Isoleucine | 4.96 | 5.38 | 5.39 |
| Leucine | 7.71 | 8.37 | 8.27 |
| Histidine | 2.39 | 2.59 | 2.44 |
| Phenylalanine | 4.66 | 5.05 | 4.76 |
| Tyrosine | 4.11 | 4.46 | 4.39 |
| Valine | 5.42 | 5.88 | 5.69 |
| Alanine | 5.35 | 5.80 | 5.76 |
| Arginine | 5.51 | 5.98 | 5.90 |
| Aspartic acid + Asparagine | 11.4 | 12.37 | 11.68 |
| Glutamic acid + Glutamine | 13.5 | 14.66 | 14.54 |
| Glycine | 4.33 | 4.70 | 4.50 |
| Proline | 3.93 | 4.27 | 3.84 |
| Serine | 4.27 | 4.64 | 4.35 |
| Methionine | 3.40 | 3.69 | 3.22 |
| Cysteine + Cystine | 0.598 | 0.649 | 0.64 |
| Tryptophan | 1.2 | 1.30 | 1.26 |
| Sum | 95.47 | | |

## Claims

1. A krill protein hydrolysate **characterized in** having
- a protein content of greater than 85% on a dry weight basis;
- less than 5% fat on a dry weight basis;
- a fluoride content of from 0.1 to 200 mg/kg hydrolysate on a dry weight basis;
- a TMAO (trimethylamine N-oxide) content of from 0.1 to 200 mg N/100 g hydrolysate on a dry weight basis;
- a TMA (trimethylamine) content of from 0.1 to 200 mg N/100 g hydrolysate on a dry weight basis;
- a TVN (total volatile nitrogen) content of from 0.1 to 200 mg N/100 g hydrolysate on a dry weight basis;
- a sodium content of from 0.01 to 4.0 g/100 g hydrolysate on a dry weight basis; and
- a calcium content of from 100 to 25,000 mg / kg hydrolysate on a dry weight basis.

2. The krill protein hydrolysate of claim 1, wherein the krill protein hydrolysate has a fluoride content of from 0.1 to 30 mg/kg on a dry weight basis.

3. The krill protein hydrolysate of any one of claims 1 to 2, wherein the krill protein hydrolysate has a TMAO content of from 0.1 to 10 mg N/100 g hydrolysate on a dry weight basis.

4. The krill protein hydrolysate of any one of claims 1 to 3, wherein the krill protein hydrolysate has a TMA content of from 0.1 to 30 mg N/100 g hydrolysate on a dry weight basis.

5. The krill protein hydrolysate of any one of claims 1 to 4, wherein the krill protein hydrolysate has a TVN content of from 0.1 to 60 mg N/100 g hydrolysate on a dry weight basis.

6. The krill protein hydrolysate of any one of claims 1 to 5, wherein the krill protein hydrolysate is further **characterized in** having a protein content of greater than 89% on a dry weight basis and a fat content of less than 2% on a dry weight basis.

7. The krill protein hydrolysate of any one of claims 1 to 6, wherein the krill protein hydrolysate has a moisture content of less than 3%.

8. The krill protein hydrolysate of any one of claims 1 to 7, wherein the krill protein hydrolysate is greater than 80% soluble in water as assayed by dissolving 4.5 g of the krill protein hydrolysate dry weight in 100 g water.

9. The krill protein hydrolysate of any one of claims 1 to 8, wherein a heat-treated protein hydrolysate is greater than 60% soluble in water as assayed by dissolving 4.5 g of the protein hydrolysate dry weight in 100 g water and heating to 85°C for five minutes.

10. The krill protein hydrolysate of any one of claims 1 to 9, wherein the krill protein hydrolysate is **characterized in** having a protein content of greater than 90%, 91%, 92%, 93%, 94% or 95% on a dry weight basis and/or a fat content of less than 3%, 2% or 1% on a dry weight basis.

11. A process for producing a filtered krill protein hydrolysate comprising:
- providing a krill meal,
- milling the krill meal,
- washing the milled krill meal with water and/or citric acid to provide a washed krill meal,
- treating the washed krill meal with a protease to provide a krill protein hydrolysate, and
- filtering the krill protein hydrolysate by microfiltration with a filter having a pore size of from 0.1 to 10 µm and/or nanofiltration with a filter having a pore size of from 1 to 10 nm to provide a filtered krill protein hydrolysate and a permeate.

12. The process of claim 11, wherein the meal is a solvent-extracted krill meal.

13. The process of any one of claims 11 to 12, further comprising a step of deactivating the protease prior to filtration.

14. The process of any one of claims 11 to 13, further comprising a step of drying the filtered krill protein hydrolysate to provide a dried krill protein hydrolysate.

15. A filtered krill protein hydrolysate made by the process of any one of claims 11 to 14.

## Patentansprüche

1. Krillproteinhydrolysat, **dadurch gekennzeichnet, dass** es aufweist:
- einen Proteingehalt von mehr als 85 % auf Trockengewichtsbasis;
- weniger als 5 % Fett auf Trockengewichtsbasis;
- einen Fluoridgehalt von 0,1 bis 200 mg/kg Hydrolysat auf Trockengewichtsbasis;
- einen TMAO(Trimethylamin-N-oxid)-Gehalt von 0,1 bis 200 mg N/100 g Hydrolysat auf Trockengewichtsbasis;
- einen TMA(Trimethylamin)-Gehalt von 0,1 bis 200 mg N/100 g Hydrolysat auf Trockengewichtsbasis;
- einen TVN(Gesamt-Flüchtigen-Stickstoff)-Gehalt von 0,1 bis 200 mg N/100 g Hydrolysat auf Trockengewichtsbasis;
- einen Natriumgehalt von 0,01 bis 4,0 g/100 g Hydrolysat auf Trockengewichtsbasis; und
- einen Calciumgehalt von 100 bis 25.000 mg/kg Hydrolysat auf Trockengewichtsbasis.

2. Krillproteinhydrolysat nach Anspruch 1, wobei das Krillproteinhydrolysat einen Fluoridgehalt von 0,1 bis 30 mg/kg auf Trockengewichtsbasis aufweist.

3. Krillproteinhydrolysat nach einem der Ansprüche 1 bis 2, wobei das Krillproteinhydrolysat einen TMAO-Gehalt von 0,1 bis 10 mg N/100 g Hydrolysat auf Trockengewichtsbasis aufweist.

4. Krillproteinhydrolysat nach einem der Ansprüche 1 bis 3, wobei das Krillproteinhydrolysat einen TMA-Gehalt von 0,1 bis 30 mg N/100 g Hydrolysat auf Trockengewichtsbasis aufweist.

5. Krillproteinhydrolysat nach einem der Ansprüche 1 bis 4, wobei das Krillproteinhydrolysat einen TVN-Gehalt von 0,1 bis 60 mg N/100 g Hydrolysat auf Trockengewichtsbasis aufweist.

6. Krillproteinhydrolysat nach einem der Ansprüche 1 bis 5, wobei das Krillproteinhydrolysat ferner **dadurch gekennzeichnet ist, dass** es einen Proteingehalt von mehr als 89 % auf Trockengewichtsbasis und einen Fettgehalt von weniger als 2 % auf Trockengewichtsbasis aufweist.

7. Krillproteinhydrolysat nach einem der Ansprüche 1 bis 6, wobei das Krillproteinhydrolysat einen Feuchtigkeitsgehalt von weniger als 3 % aufweist.

8. Krillproteinhydrolysat nach einem der Ansprüche 1 bis 7, wobei das Krillproteinhydrolysat zu mehr als 80 % in Wasser löslich ist, wie durch Lösen von 4,5 g des Krillproteinhydrolysats (Trockengewicht) in 100 g Wasser bestimmt wird.

9. Krillproteinhydrolysat nach einem der Ansprüche 1 bis 8, wobei ein wärmebehandeltes Proteinhydrolysat zu mehr als 60 % in Wasser löslich ist, wie durch Lösen von 4,5 g des Proteinhydrolysats (Trockengewicht) in 100 g Wasser und fünfminütiges Erwärmen auf 85 C bestimmt wird.

10. Krillproteinhydrolysat nach einem der Ansprüche 1 bis 9, wobei das Krillproteinhydrolysat **dadurch gekennzeichnet ist, dass** es einen Proteingehalt von mehr als 90 %, 91 %, 92 %, 93 %, 94 % oder 95 % auf Trockengewichtsbasis und/oder einen Fettgehalt von weniger als 3 %, 2 % oder 1 % auf Trockengewichtsbasis aufweist.

11. Verfahren zur Herstellung eines filtrierten Krillproteinhydrolysats, umfassend:
- Bereitstellen eines Krillmehls,
- Mahlen des Krillmehls,
- Waschen des gemahlenen Krillmehls mit Wasser und/oder Zitronensäure, um ein gewaschenes Krillmehl bereitzustellen,
- Behandeln des gewaschenen Krillmehls mit einer Protease, um ein Krillproteinhydrolysat bereitzustellen, und
- Filtrieren des Krillproteinhydrolysats durch Mikrofiltration mit einem Filter, der eine Porengröße von 0,1 bis 10 µm aufweist, und/oder Nanofiltration mit einem Filter, der eine Porengröße von 1 bis 10 nm aufweist, um ein filtriertes Krillproteinhydrolysat und ein Permeat bereitzustellen.

12. Verfahren nach Anspruch 11, wobei das Mehl ein lösungsmittelextrahiertes Krillmehl ist.

13. Verfahren nach einem der Ansprüche 11 bis 12, das ferner einen Schritt der Deaktivierung der Protease vor der Filtration umfasst.

14. Verfahren nach einem der Ansprüche 11 bis 13, das ferner einen Schritt des Trocknens des filtrierten Krillproteinhydrolysats umfasst, um ein getrocknetes Krillproteinhydrolysat bereitzustellen.

15. Filtriertes Krillproteinhydrolysat, hergestellt durch das Verfahren nach einem der Ansprüche 11 bis 14.

## Revendications

1. Hydrolysat de protéines de krill **caractérisé en ce qu'**il présente
- une teneur en protéines supérieure à 85 % sur une base en poids sec ;
- moins de 5 % de matières grasses sur une base en poids sec ;
- une teneur en fluorure de 0,1 à 200 mg/kg d'hydrolysat sur une base en poids sec ;
- une teneur en TMAO (triméthylamine-N-oxyde) de 0,1 à 200 mg N/100 g d'hydrolysat sur une base en poids sec ;
- une teneur en TMA (triméthylamine) de 0,1 à 200 mg N/100 g d'hydrolysat sur une base en poids sec ;
- une teneur en TVN (azote volatil total) de 0,1 à 200 mg N/100 g d'hydrolysat sur une base en poids sec ;
- une teneur en sodium de 0,01 à 4,0 g/100 g d'hydrolysat sur une base en poids sec ; et
- une teneur en calcium de 100 à 25 000 mg/kg d'hydrolysat sur une base en poids sec.

2. Hydrolysat de protéines de krill selon la revendication 1, dans lequel l'hydrolysat de protéines de krill a une teneur en fluorure de 0,1 à 30 mg/kg sur une base en poids sec.

3. Hydrolysat de protéines de krill selon l'une quelconque des revendications 1 à 2, dans lequel l'hydrolysat de protéines de krill a une teneur en TMAO de 0,1 à 10 mg N/100 g d'hydrolysat sur une base en poids sec.

4. Hydrolysat de protéines de krill selon l'une quelconque des revendications 1 à 3, dans lequel l'hydrolysat de protéines de krill a une teneur en TMA de 0,1 à 30 mg N/100 g d'hydrolysat sur une base en poids sec.

5. Hydrolysat de protéines de krill selon l'une quelconque des revendications 1 à 4, dans lequel l'hydrolysat de protéines de krill a une teneur en TVN de 0,1 à 60 mg N/100 g d'hydrolysat sur une base en poids sec.

6. Hydrolysat de protéines de krill selon l'une quelconque des revendications 1 à 5, dans lequel l'hydrolysat de protéines de krill est en outre **caractérisé en ce qu'**il a une teneur en protéines supérieure à 89 % sur une base en poids sec et une teneur en matière grasse inférieure à 2 % sur une base en poids sec.

7. Hydrolysat de protéines de krill selon l'une quelconque des revendications 1 à 6, dans lequel l'hydrolysat de protéines de krill a une teneur en humidité inférieure à 3 %.

8. Hydrolysat de protéines de krill selon l'une quelconque des revendications 1 à 7, dans lequel l'hydrolysat de protéines de krill est soluble dans l'eau à plus de 80 % tel que testé par dissolution de 4,5 g de poids sec d'hydrolysat de protéines de krill dans 100 g d'eau.

9. Hydrolysat de protéines de krill selon l'une quelconque des revendications 1 à 8, dans lequel un hydrolysat de protéines thermotraité est soluble dans l'eau à plus de 60 % tel que testé par dissolution de 4,5 g de poids sec d'hydrolysat de protéines dans 100 g d'eau et chauffage à 85 °C pendant 5 minutes.

10. Hydrolysat de protéines de krill selon l'une quelconque des revendications 1 à 9, dans lequel l'hydrolysat de protéines de krill est **caractérisé en ce qu'**il a une teneur en protéines supérieure à 90 %, 91 %, 92 %, 93 %, 94 % ou 95 % sur une base en poids sec et/ou une teneur en matières grasses inférieure à 3 %, 2 % ou 1 % sur une base en poids sec.

11. Procédé de production d'un hydrolysat de protéines de krill filtré comprenant :
- la fourniture d'une farine de krill,
- le broyage de la farine de krill,
- le lavage de la farine de krill broyée avec de l'eau et/ou de l'acide citrique pour fournir une farine de krill lavé,
- le traitement de la farine de krill lavée avec une protéase pour fournir un hydrolysat de protéines de krill, et
- la filtration de l'hydrolysat de protéines de krill par microfiltration avec un filtre ayant une taille de pores de 0,1 à 10 µm et/ou par nanofiltration avec un filtre ayant une taille de pores de 1 à 10 nm pour fournir un hydrolysat de protéines de krill filtré et un perméat.

12. Procédé selon la réalisation 11, dans lequel la farine est une farine de krill extraite par solvant.

13. Procédé selon l'une quelconque des revendications 11 à 12, comprenant en outre une étape de désactivation de la protéase avant la filtration.

14. Procédé selon l'une quelconque des revendications 11 à 13, comprenant en outre une étape de séchage de l'hydrolysat de protéines de krill filtré pour fournir un hydrolysat de protéines de krill sec.

15. Hydrolysat de protéines de krill filtré préparé par le procédé selon l'une quelconque des revendications 11 à 14.
